# EUROPEAN PATENT APPLICATION

(11) **EP 4 445 904 A1**
(43) Date of publication of application: **16.10.2024**
(21) Application number: 24167287.2
(22) Date of filing: 28.03.2024
(51) Int. Cl.: A61K 33/30, A61K 31/192, A61P 15/02, A61K 9/06, A61K 9/00

(54) **PHARMACEUTICAL COMPOSITION FOR TREATING OR PREVENTING VULVOAGINAL CANDIDIASIS**

(30) Priority: 31.03.2023 US 202363456387 P
(71) Applicant: Fempharma, LLC, 4029 Debrecen (HU)
(72) Inventor: TAKACS, Peter, H-4029 Debrecen (HU)
(74) Representative: D Young & Co LLP

(57) **Abstract**

Provided are a zinc-containing pharmaceutical composition, preferably in the form of a hydrogel; a kit comprising the composition; and methods of treating or preventing vulvovaginal candidiasis by administering the composition to a subject in need thereof.

## Description

### BACKGROUND

One of the most frequent reasons for patient visits to obstetrician-gynecologists are vaginal symptoms secondary to infections *(1).* These symptoms have significant consequences regarding discomfort and pain, days lost from school or work, sexual functioning, and self-image *(2, 3).*

Vaginitis is an inflammation or infection of the vagina and is associated with a spectrum of symptoms, including vulvovaginal itching, burning, irritation, dyspareunia, "fishy" vaginal odor, and abnormal vaginal discharge *(3).* Vaginitis is very common in adult women throughout their lifespans.

An estimated 7.4 million new cases of bacterial vaginosis occur only in the US annually, which accounts for 40-50% of vaginitis cases. Innovative treatments and the extensive research of vaginal microbiome are evolving; however, vaginitis remains challenging for clinicians. Although the prognosis is excellent, recurrent vaginal infections can lead to chronic irritation, sexual dysfunction, and consequent psychosocial and emotional stress. In addition, recurrent vaginitis is associated with a growing list of significant morbidities, especially preterm labor, acquisition of sexually transmitted infections, and enhanced HIV transmission *(4).*

After bacterial vaginosis, vulvovaginal candidiasis is the second most common cause of vaginitis symptoms and covers approximately one-third of vaginitis cases *(20).* According to *Foxman et al.,* 29-49% of premenopausal women report having had at least one lifetime episode, and 9% of women report having RVVC in 12 months *(21).* RVVC is defined as three or more episodes of symptomatic infection within one year *(22).*

There are many gaps in our knowledge of the pathogenesis of RVVC. In most cases, RVVC remains idiopathic in its origin. However, contributing factors, such as altered immune response and genetic polymorphisms, have been identified, and potential mechanisms are under study. According to *Sobel,* there is currently no mechanism or vehicle to control the host mucosal reaction other than by facilitating *Candida* antigen tolerance by keeping the vaginal fungal load at markedly reduced levels *(22).*

One-third of women with an initial response to therapy for vaginitis have a recurrence within three months, whereas 58% recur within 12 months *(3).* Multiple etiologies likely contribute to the high recurrence rates, such as inadequate treatment, reinfection and infection relapse, genetics, and antimicrobial resistance. Even though antimicrobial resistance has not been systematically studied or documented, patient-based studies provide evidence of progressive antimicrobial resistance *(5, 6).* Any additional advance in the therapy of vaginitis requires new therapeutic approaches to this common syndrome.

### SUMMARY OF THE INVENTION

Some of the main aspects of the present invention are summarized below. Additional aspects are described in the Detailed Description of the Invention, Examples, Drawings, and Claims sections of this disclosure. The description in each section of this disclosure is intended to be read in conjunction with the other sections. Furthermore, the various embodiments described in each section of this disclosure can be combined in various different ways, and all combinations of disclosed embodiments are intended to fall within the scope of the present invention.

In one aspect, the present study demonstrates that prophylactic use of zinc-containing vaginal hydrogel decreases the recurrence rate of vaginal infections in women diagnosed with recurrent vaginal infections. Use of the zinc-containing vaginal hydrogel resulted in a 62% reduction in infections. Unexpectedly, the zinc-containing gel was highly effective in women with RVVC, but not in women with RBV. In particular, women with a history of RVVC had an 83% reduction in symptomatic vaginal infections using the zinc-containing vaginal gel, while both of the women diagnosed with RBV experienced a recurrent infection.

Accordingly, the present invention provides a pharmaceutical composition comprising about 2-200 µM zinc, for use in a method of treating or preventing vulvovaginal candidiasis (VVC) in a subject having VVC or susceptible to VVC, wherein the composition is formulated for vaginal delivery. Additionally provided is a pharmaceutical composition comprising about 2-200 µM zinc, for use in a method of treating recurrent vulvovaginal candidiasis (RVVC) in a subject susceptible to RVVC, wherein the composition is formulated for vaginal delivery.

In one embodiment, the invention provides a method of treating or preventing vulvovaginal candidiasis (VVC) in a subject having VVC or susceptible to VVC, the method comprising vaginally administering to the subject a composition comprising about 2-200 µM zinc, wherein administration is at least once daily for at least one week.

In some embodiments, the subject has VVC. In some embodiments, the subject has experienced recurrent VVC.

In another aspect, provided is a method of treating recurrent vulvovaginal candidiasis (RVVC) in a subject susceptible to RVVC, the method comprising vaginally administering to the subject a composition comprising about 2-200 µM zinc, wherein administration is at least once daily for at least one week.

In a particular embodiment, the composition comprises about 20 µM zinc. The zinc can be in any suitable form, such as in the form of zinc sulfate. In one embodiment, the composition comprises zinc sulfate heptahydrate.

In certain embodiments, the pharmaceutical composition is in the form of a hydrogel. The hydrogel can comprise, for example, a hydroxyethyl-cellulose (HEC) polymer. In a particular embodiment, the composition is an HEC polymer.

In one embodiment, the pharmaceutical composition comprises lactic acid.

In certain embodiments, administration is once daily or twice daily or twice weekly or three times weekly. In particular embodiments, administration is for a total of at least two weeks, or at least six weeks, or at least two months, or at least three months.

In one embodiment, administration is at least once daily for at least one week.

In a particular embodiment, administration is at least once daily for at least two weeks.

In a particular embodiment, administration is once daily for two weeks, followed by at least twice per week for at least two weeks. In a further embodiment, administration is once daily for two weeks, followed by at least twice per week for at least six weeks.

In a particular embodiment, administration is once daily for two weeks, followed by at least twice per week up to a total period of at least two months.

In a particular embodiment, administration is once daily for two weeks, followed by at least twice per week up to a total period of at least three months.

Preferably, administration is self-administration.

A kit comprising the pharmaceutical composition and instructions for use is also provided.

Another aspect of the invention is the use of a hydrogel comprising about 2-200 µM zinc in the manufacture of a medicament for treating or preventing vulvovaginal candidiasis (VVC) or recurrent VVC (RVVC) in a subject having VVC or susceptible to VVC or RVCC, wherein the medicament is formulated for vaginal delivery.

### BRIEF DESCRIPTION OF THE DRAWING

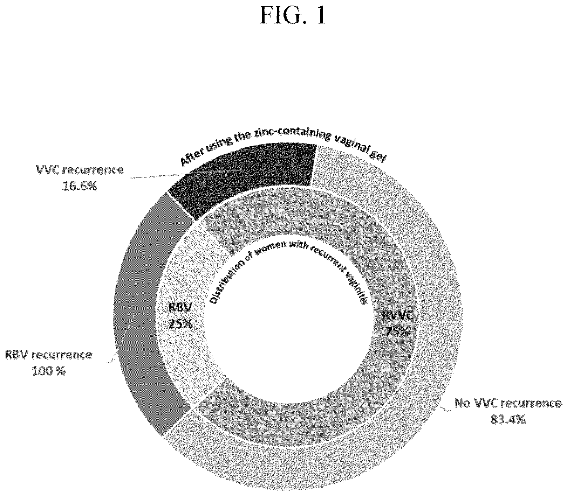

**FIG. 1** shows the effect of the zinc-containing vaginal gel on repeat infection in women with a history of recurrent vaginal infections. Out of eight women, six were diagnosed with RVVC (75%) and two with RBV (25%). One out of six RVVC patients (16.6%) and two out of two RBV patients (100%) develop vaginitis during three months of treatment with the zinc-containing vaginal gel. Women with a history of RVVC had an 83% reduction in symptomatic vaginal infections using the zinc-containing vaginal gel (P=0.004).

### DETAILED DESCRIPTION OF THE INVENTION

Unless defined otherwise, all technical and scientific terms used herein have the same meaning as commonly understood by one of ordinary skill in the art to which this invention is related.

As used in this specification and the appended claims, the singular forms "a," "an," and "the" include plural referents, unless the context clearly dictates otherwise. The terms "a" (or "an") as well as the terms "one or more" and "at least one" can be used interchangeably.

Furthermore, "and/or" is to be taken as specific disclosure of each of the two specified features or components with or without the other. Thus, the term "and/or" as used in a phrase such as "A and/or B" is intended to include A and B, A or B, A (alone), and B (alone). Likewise, the term "and/or" as used in a phrase such as "A, B, and/or C" is intended to include A, B, and C; A, B, or C; A or B; A or C; B or C; A and B; A and C; B and C; A (alone); B (alone); and C (alone).

Units, prefixes, and symbols are denoted in their Système International de Unites (SI) accepted form. Numeric ranges are inclusive of the numbers defining the range. Where a numeric term is preceded by "about," the term includes the stated number and values ±10% of the stated number. The headings provided herein are not limitations of the various aspects or embodiments of the invention, which can be had by reference to the Specification as a whole. Accordingly, the terms defined immediately below are more fully defined by reference to the specification in its entirety.

Wherever embodiments are described with the language "comprising," otherwise analogous embodiments described in terms of "consisting of" and/or "consisting essentially of" are included.

An "effective amount" of an active agent or a pharmaceutical composition as disclosed herein is an amount sufficient to carry out a specifically stated purpose, in relation to the route of administration and dosage form.

An "active agent" is an agent which itself has biological activity, or which is a precursor or prodrug that is converted in the body to an agent having biological activity.

The term "pharmaceutical composition" refers to a collection of ingredients or substituents in such form as to permit the biological activity of the active ingredient(s) to be effective, and which contains no additional components that are unacceptably toxic to a subject to which the composition would be administered. In embodiments in which more than one active agent is administered, the agents can be administered together (for example, in the same formulation and/or at the same time), or separately (for example, in different formulations and/or at different times). Accordingly, a pharmaceutical composition of the invention can comprise one or multiple dosage units.

Pharmaceutical compositions generally can be in numerous dosage forms or combinations of dosage forms. Provided herein is a pharmaceutical composition formulated for local administration to the vulvovaginal region. In certain embodiments, the composition can be, for example, a cream, a lotion, a paste, a gel, a mousse, a foam, a lacquer, a suspension, a liquid, a spray, a suppository, or a capsule. In a preferred embodiment, the composition is a hydrogel.

Hydrogels are a class of polymers made from hydrophilic repeat units that interact with water molecules by hydrogen bonding and polar and ionic interaction to take up water many times the initial polymer weight *(14).* Hydrogels are hydrophilic solid materials that do not dissolve in an aqueous medium, with a three-dimensional, crosslinked molecular structure that absorbs large quantities of water and swells in a physiological environment. Wichterle and Lim published a seminal paper in Nature in 1960, proposing that a biocompatible material should have a molecular structure affording the desired water content, be inert to normal biological processes, and have permeability to metabolites *(26).* Hydrogels have evolved significantly since that time.

Hydrogels are employed in drug delivery, diapers, cosmetics, plastic surgery, vaccination, cancer therapy, water purification, cultivation of micro-organisms, and tissue repair and regeneration *(27).* Hydrogels have many advantages in drug delivery, principally their biocompatibility potential, hydrophilicity, controlled drug release, and smart drug delivery *(28).* A recent study revealed that a chitosan-based hydrogel is a viable option for the vaginal administration of progesterone *(15)*; inert and active hydrogels also have great importance in burn first aid and infected wound treatment *(16, 17)*.

Among hydrogels, zinc-containing hydrogels offer emerging possibilities in health sciences. Their range of use extends from infected wound treatment to hydrogels with hemostatic activities or zinc-containing vaginal moisturizer gel *(13*, *17*, *18).* Although several vaginal hydrogels are commercially available over the counter as lubricants or moisturizers, very few clinical trials have been performed to show their safety and efficacy *(19).*

In one embodiment, the pharmaceutical composition of the invention is a hydrogel comprising a hydroxyethyl-cellulose (HEC) polymer, zinc, and lactic acid. Lactic acid provides the appropriate acidic pH for maintaining the required vaginal environment favored by lactobacilli. The pH of the pharmaceutical composition is ideally between about 3.5 and 5.5, including for example, about 4.0, about 4.5, or about 5.0, and ranges with any of the aforementioned endpoints. In a particular embodiment, the pH of the composition is about 4.4.

HEC is a water-soluble and biocompatible cellulose derivative with many hydroxy groups in its structure. It has the advantage of being modified and improved by grafting polymerization with vinyl groups *(29).* HEC-based hydrogels can be ideal biomaterials for zinc-delivering drugs because they can have a maximum biological response while keeping the amount of zinc to a minimum. In addition, HEC polymer can bind to the vaginal mucosa and keep moisture on the epithelial surface.

Zinc, an essential trace element in the human body, plays critical roles in human growth, development, and innate and adaptive immune responses *(7*, *23).* Zinc is crucial in preventing infections; zinc deficiency is closely linked to impaired mucosal integrity *(8).* In addition, the presence of zinc in the epidermis promotes epidermal homeostasis *(9).*

*In vitro* studies suggest that zinc might play a positive role in antagonizing *Candida albicans* pathogenicity. Zinc limitation has recently been reported to induce a hyper-adherent phenotype in *Candida albicans (24).* Citiulo *et al.* report that the pH-regulated antigen 1 (Pra1) of *Candida albicans* binds zinc from its environment *(25).* The deletion of PRA1 prevented the utilization of host zinc and damage to host cells in the absence of exogenous zinc *(25).* Pra1 is strongly repressed at acidic pH *(25).*

Oral supplementation with zinc has no significant impact on cervicovaginal lavage zinc level, despite a significant rise in serum zinc level *(10).* However, vaginal application of zinc in the form of a zinc-containing vaginal gel is a viable option for delivering zinc to the vagina *(11)*. Zinc transporters are present in the vagina, and ZIP4 is present in the vaginal wall for absorption of vaginal zinc replenishment *(12).*

We previously investigated the effect of zinc-containing vaginal hydrogel on postmenopausal vulvovaginal symptoms, and showed that the hydrogel significantly improves postmenopausal vulvovaginal symptoms, such as vaginal dryness and dyspareunia, without serious side effects *(13).*

The pharmaceutical composition of the invention can comprise at total of about 2-200 µM zinc, a total of at least about 2, 5, 10, 15, 20, 25, 30, 35, 40, 45, 50, 55, 60, 65, 70, 75, 80, 85, 90, 95, 100, 120, 140, 160, 180, or 200 µM zinc, or any dosage range of zinc having these amounts as endpoints. In one embodiment, the amount of zinc is about 20 µM. The zinc can be in any suitable form including, for example, as acetate, citrate, gluconate, orotate, picolinate, or sulfate. In a particular embodiment, the composition comprises a zinc sulfate, such as zinc sulfate heptahydrate. The zinc sulfate heptahydrate complex binds seven water molecules, providing the necessary hydration to the vagina.

By "subject" or "individual" or "patient" is meant a mammalian subject, for whom diagnosis, prognosis, therapy, or prevention is desired. In one embodiment, the subject is a human.

The subject may suffer from or be at risk of developing vulvovaginal candidiasis (VVC), including recurrent vulvovaginal candidiasis (RVVC).

In some embodiments, the invention provides a method of treating VVC in a subject having VVC or of preventing VVC in a subject susceptible to VVC. In one embodiment, the subject is susceptible to or at risk of developing RVVC. One embodiment provides a method of treating RVVC in a subject susceptible to RVVC.

Terms such as "treating" or "treatment" or "to treat" or "alleviating" or "to alleviate" refer to therapeutic measures that cure, slow down, lessen symptoms of, and/or halt progression of a diagnosed pathologic condition or disorder. Thus, those in need of treatment include those already with or having experienced the disorder. In certain embodiments, a subject is successfully "treated" for a disease or disorder according to the methods provided herein if the patient shows, *e.g*., total, partial, or transient alleviation or elimination of symptoms associated with the disease or disorder; diminishment of the extent or frequency of the condition, disease, or disorder; stabilization (*i.e*., not worsening) of the condition, disease, or disorder; or slowing of progression of the condition, disease, or disorder.

"Prevent" or "prevention" refers to prophylactic or preventative measures that prevent and/or slow the development of a targeted pathologic condition or disorder. Thus, those in need of prevention include those at risk of or susceptible to developing the disorder. Subjects that are at risk of or susceptible to developing VVC include, but are not limited to, women who have experienced RVVC. Other risk factors include use of oral antibiotics, use of birth control pills, sexual activity, diabetes, history of sexually transmitted diseases, vaginal douching, impaired immune system, and hormonal changes, such as those associated with pregnancy. In certain embodiments, a disease or disorder is successfully prevented according to the methods provided herein if the patient develops, transiently or permanently, *e.g*., fewer or less severe symptoms associated with the disease or disorder, or a later onset of symptoms associated with the disease or disorder, than a patient who has not been subject to the methods of the invention.

VVC can be assessed using a variety of measures, including clinical examination, potassium hydroxide preparation, and/or fungal culture.

Patients subjected to methods of the present invention can demonstrate clinical signs of VVC, including one or more of vaginal erythema, edema, excoriation, or discharge, and/or symptoms of VVC, including vaginal itching, burning, irritation, or discharge.

Alternatively, improvement can be assessed relative to a control population. For example, incidence of RVVC in a subject susceptible to RVVC and receiving the pharmaceutical composition of the invention can be compared with the average incidence of RVVC in subjects susceptible to RVVC who did not receive the pharmaceutical composition of the invention. Likewise, improvement in degree and/or length of symptoms of VVC in a subject receiving the pharmaceutical composition of the invention can be compared with the average improvement of symptoms in subjects with VVC who did not receive the pharmaceutical composition of the invention. Subjects in the control population may be treated by conventional methods, such as antifungal compositions.

The pharmaceutical composition can be administered according to any suitable dosing regimen, for example, where the daily dose is divided into two or more separate doses. It is within the skill of the ordinary artisan to determine a dosing schedule and duration. In some embodiments, the pharmaceutical composition is administered intravaginally or topically to the vulvovaginal region or once a day. Typically, administration is self-administration.

The composition is administered at least once daily for at least one week, preferably for at least two weeks. In some embodiments, the composition is further administered at least once weekly, preferably at least twice weekly, after the initial period of daily administration. In some embodiments, the composition is administered for at total of at least about 1, 2, 3, 4, 5, 6, 7, 8, 9, 10, 11, 12, or 13 weeks, or for at least about 1, 2, 3, 4, 5, or 6 months.

Also within the scope of the invention are kits comprising the pharmaceutical composition provided herein and instructions for use in the treatment or prevention of VVC and/or RVVC. Kits typically include a label indicating the intended use of the contents of the kit. The term "label" includes any writing, or recorded material supplied on or with the kit, or which otherwise accompanies the kit. The label may include, for example, instructions for administration.

A kit of the invention generally comprises multiple doses of the pharmaceutical composition. In some embodiments, each dose is divided into individual dosage forms, for example, in a tube, pouch, blister pack, suppository, or prefilled applicator.

All of the references cited in this disclosure are hereby incorporated by reference in their entireties. In addition, any manufacturers' instructions or catalogues for any products cited or mentioned herein are incorporated by reference. Documents incorporated by reference into this text, or any teachings therein, can be used in the practice of the present invention. Documents incorporated by reference into this text are not admitted to be prior art.

Embodiments of the present disclosure can be further defined by reference to the following non-limiting examples. It will be apparent to those skilled in the art that many modifications, both to materials and methods, can be practiced without departing from the scope of the present disclosure.

### EXAMPLES

### Example 1. Pilot Study of Zinc-Containing Hydrogel for Vaginal Infection

### Study Methods and Results

A retrospective cohort study was performed after reviewing the medical records of women with a history of recurrent vaginal infections [recurrent bacterial vaginosis (RBV) or recurrent vulvovaginal candidiasis (RVVC)], who used a zinc-containing vaginal hydrogel as a prophylactic treatment. The study was carried out at the outpatient clinic of the Department of Obstetrics and Gynecology, University of Debrecen, Hungary.

Enrollment criteria included the presence of RBV or RVVC. Recurrent vaginal infection was defined as three or more vaginal infections in the last 12 months *(3).* During the initial evaluation, the differential diagnosis of BV and VVC was established by obtaining a history and performing a physical examination, which encompassed a speculum examination of the vagina and analysis of the nature of the discharge and additional symptoms. Individuals whose initial evaluation confirmed the diagnosis were then enrolled in the study. Exclusion criteria were a postmenopausal state, defined as individuals had to have at least 12 consecutive months of amenorrhea without any other obvious reason; consistently elevated follicle-stimulating hormone blood levels of 30 mIU/mL or higher and local or systemic hormone therapy within the past six months; cytological atypia; prior radiation treatment; history of breast, ovarian or other gynecological cancer; pelvic organ prolapse > stage 2; or use within the prior 3 months of any vaginal product or douching.

Patients underwent a detailed gynecological exam at enrollment and a follow-up visit. At the first visit, general gynecological and medical history was taken, including age, body mass index (BMI), previous pregnancies, deliveries or operations, menstruation cycle, hormonal therapy, current relationship status, sexual partners in the last 12 months, and episodes of vaginitis over the previous 12 months.

Ten women were enrolled. Two were lost to follow-up, and eight completed the study. Demographic and pertinent clinical information was recorded and stored in a dedicated database. A summary is shown in **Table 1.**

**Table 1. Clinical and Demographic Characteristics of Study Subject**

| **Variable** | **Data** |
|---|---|
| Subj ects | 10 (100) |
| Age [years (mean ± SD)] | 32 ± 6 |
| BMI [kg/m² (mean ± SD)] | 24 ± 5 |
| Gravida [mean (range)] | 1.5 (0-3) |
| Parity [mean (range)] | 1.5 (0-3) |
| Number of sexual partners [mean (range)] | 1 (1-3) |

| | |
|---|---|
| BMI: Body Mass Index; Gravida: number of pregnancies, Parity: number of deliveries | |

Women self-administered a zinc-containing vaginal hydrogel comprising water, hydroxyethyl cellulose, zinc sulfate, and lactic acid, as a prophylactic treatment. The novel hydrogel was applied intravaginally via a vaginal applicator. Participants placed 2 mL of gel into the vagina daily for two consecutive weeks and, twice per week subsequently, through the conclusion of the study. Women were asked to return to the clinic for evaluation if any symptoms of vaginal infection were present.

Statistical analysis was performed with Microsoft Excel 2019 (Microsoft, Redmond, WA, USA). To describe the clinical and demographic characteristics, means ± standard deviation (SD) or means (range) were used for continuous variables. Paired t-test was used to compare differences in the number of recurrent and non-recurrent subjects. Differences were considered significant when the P value was less than 0.05.

Based on the initial evaluation, of the eight participants who completed the study, six were diagnosed with RVVC and two with RBV. On average, there was at least one infection every three months before treatment.

After the zinc-containing vaginal gel treatment, 5 out of 8 women did not have an infection in the first three months (*P*=0.04). Three women, one with RVVC and two with RBV, developed one vaginal infection resulting in a 62% reduction in infections (**FIG. 1**). Women with a history of RVVC had an 83% reduction in symptomatic vaginal infections using the zinc-containing vaginal gel (*P*=0.004).

### REFERENCES

1. M. R. Anderson, K. Klink, A. Cohrssen, Evaluation of vaginal complaints. Jama. 291, 1368-1379 (2004).
2. Y. Zhu et al., Health-related quality of life as measured with the Short-Form 36 (SF-36) questionnaire in patients with recurrent vulvovaginal candidiasis. Health. Qual. Life. Outcomes. 14, 65-016-0470-2 (2016).
3. Vaginitis in Nonpregnant Patients: ACOG Practice Bulletin, Number 215. Obstet. Gynecol. 135, e1-e17 (2020).
4. S. L. Hillier et al., Association between bacterial vaginosis and preterm delivery of a low-birth-weight infant. The Vaginal Infections and Prematurity Study Group. N. Engl. J. Med. 333, 1737-1742 (1995).
5. A. Mollin, M. Katta, J. D. Sobel, R. A. Akins, Association of key species of vaginal bacteria of recurrent bacterial vaginosis patients before and after oral metronidazole therapy with short- and long-term clinical outcomes. PLoS One. 17, e0272012 (2022).
6. J. M. Marrazzo, K. K. Thomas, T. L. Fiedler, K. Ringwood, D. N. Fredricks, Relationship of specific vaginal bacteria and bacterial vaginosis treatment failure in women who have sex with women. Ann. Intern. Med. 149, 20-28 (2008).
7. E. Goethe et al., Critical Role of Zur and SmtB in Zinc Homeostasis of Mycobacterium smegmatis. mSystems. 5, 10.1128/mSystems.00880-19 (2020).
8. W. Ohashi, T. Fukada, Contribution of Zinc and Zinc Transporters in the Pathogenesis of Inflammatory Bowel Diseases. J. Immunol. Res. 2019, 8396878 (2019).
9. Y. Ogawa, M. Kinoshita, S. Shimada, T. Kawamura, Zinc in Keratinocytes and Langerhans Cells: Relevance to the Epidermal Homeostasis. J. Immunol. Res. 2018, 5404093 (2018).
10. P. Takacs, P. Damjanovich, A. G. Sipos, B. Kozma, The effect of oral zinc supplementation on cervicovaginal lavage fluid zinc level. Eur. J. Obstet. Gynecol. Reprod. Biol. 248, 106-109 (2020).
11. F. Fenyvesi et al., Biocompatibility and zinc release testing of a zinc-containing vaginal gel. Menopause. 27, 143-149 (2020).
12. A. Csikos et al., Zinc Transporter 9 (SLC30A9) Expression Is Decreased in the Vaginal Tissues of Menopausal Women. Biol. Trace Elem. Res. 199, 4011-4019 (2021).
13. P. Takacs et al., Zinc-containing Vaginal Moisturizer Gel Improves Postmenopausal Vulvovaginal Symptoms: A Pilot Study. J. Menopausal Med. 25, 63-68 (2019).
14. E. Jabbari, Hydrogels for Cell Delivery. Gels. 4, 10.3390/gels4030058 (2018).
15. O. Afloarea, C. N. Cheaburu Yilmaz, L. Verestiuc, N. Bibire, Development of Vaginal Carriers Based on Chitosan-Grafted-PNIPAAm for Progesterone Administration. Gels. 8, 10.3390/gels8090596 (2022).
16. A. Surowiecka, J. Struzyna, A. Winiarska, T. Korzeniowski, Hydrogels in Burn Wound Management-A Review. Gels. 8, 10.3390/gels8020122 (2022).
17. Y. Chen et al., A dZnONPs Enhanced Hybrid Injectable Photocrosslinked Hydrogel for Infected Wounds Treatment. Gels. 8, 10.3390/gels8080463 (2022).
18. C. M. Yang et al., Silk Fibroin/Tannin/ZnO Nanocomposite Hydrogel with Hemostatic Activities. Gels. 8, 10.3390/gels8100650 (2022).
19. C. S. Dezzutti et al., Is wetter better? An evaluation of over-the-counter personal lubricants for safety and anti-HIV-1 activity. PLoS One. 7, e48328 (2012).
20. K. A. Workowski, G. A. Bolan, Centers for Disease Control and Prevention, Sexually transmitted diseases treatment guidelines, 2015. MMWR Recomm Rep. 64, 1-137 (2015).
21. B. Foxman, R. Muraglia, J. Dietz, J. D. Sobel, J. Wagner, Prevalence of recurrent vulvovaginal candidiasis in 5 European countries and the United States: results from an internet panel survey. J. Low Genit Tract Dis. 17, 340-345 (2013).
22. J. D. Sobel, Recurrent vulvovaginal candidiasis. Am. J. Obstet. Gynecol. 214, 15-21 (2016).
23. N. Z. Gammoh, L. Rink, Zinc in Infection and Inflammation. Nutrients. 9, 10.3390/nu9060624 (2017).
24. D. Malavia et al., Zinc Limitation Induces a Hyper-Adherent Goliath Phenotype in Candida albicans. Front. Microbiol. 8, 2238 (2017).
25. F. Citiulo et al., Candida albicans scavenges host zinc via Pra1 during endothelial invasion. PLoS Pathog. 8, e1002777 (2012).
26. O. Wichterle, D. Lím, Hydrophilic Gels for Biological Use. Nature. 185, 117-118 (1960).
27. E. Jabbari, "Preface to Hydrogels in Tissue Engineering" in Hydrogels in Tissue Engineering (MDPI, Basel, 2018), pp. ix-x.
28. Y. Qiu, K. Park, Environment-sensitive hydrogels for drug delivery. Adv. Drug Deliv. Rev. 53, 321-339 (2001).
29. J. Wang, W. Wang, A. Wang, Synthesis, characterization and swelling behaviors of hydroxyethyl cellulose-g-poly(acrylic acid)/attapulgite superabsorbent composite. Polym. Eng. Sci. 50, 1019-1027 (2010).

The foregoing description of the specific embodiments will so fully reveal the general nature of the invention that others can, by applying knowledge within the skill of the art, readily modify and/or adapt for various applications such specific embodiments, without undue experimentation, without departing from the general concept of the present invention. Therefore, such adaptations and modifications are intended to be within the meaning and range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance. The present invention is further described by the following claims.

## Claims

1. A pharmaceutical composition comprising about 2-200 µM zinc, for use in a method of treating or preventing vulvovaginal candidiasis (VVC) in a subject having VVC or susceptible to VVC, wherein the composition is formulated for vaginal delivery.

2. The pharmaceutical composition for use according to claim 1, wherein the subject has VVC.

3. The pharmaceutical composition for use according to claim 1 or 2, wherein the subject has experienced recurrent VVC.

4. A pharmaceutical composition comprising about 2-200 µM zinc, for use in a method of treating recurrent vulvovaginal candidiasis (RVVC) in a subject susceptible to RVVC, wherein the composition is formulated for vaginal delivery.

5. The pharmaceutical composition for use according to any preceding claim, wherein the zinc is zinc sulfate, preferably zinc sulfate heptahydrate.

6. The pharmaceutical composition for use according to any preceding claim, wherein the composition comprises about 20 µM zinc.

7. The pharmaceutical composition for use according to any preceding claim, wherein the composition is a hydrogel; optionally wherein the hydrogel comprises a hydroxyethyl-cellulose (HEC) polymer, preferably wherein the hydrogel is an HEC polymer.

8. The pharmaceutical composition for use according to any preceding claim, wherein the composition comprises lactic acid.

9. The pharmaceutical composition for use according to any preceding claim, wherein administration of the pharmaceutical composition is self-administration.

10. The pharmaceutical composition for use according to any preceding claim, wherein administration of the pharmaceutical composition is at least once daily for at least one week.

11. The pharmaceutical composition for use according to any preceding claim, wherein administration of the pharmaceutical composition is once daily.

12. The pharmaceutical composition for use according to any preceding claim, wherein administration of the pharmaceutical composition is for at least two weeks.

13. The pharmaceutical composition for use according to any preceding claim, further comprising administration of the pharmaceutical composition twice per week for at least two weeks.

14. The pharmaceutical composition for use according to any preceding claim, further comprising administration of the pharmaceutical composition twice per week for at least six weeks.

15. The pharmaceutical composition for use according to any preceding claim, wherein administration of the pharmaceutical composition is for a total of at least two months, preferably for at least three months.
